# EUROPEAN PATENT SPECIFICATION

(11) **EP 2 088 914 B1**
(45) Date of publication and mention of the grant of the patent: **16.02.2011**
(21) Application number: 07830714.7
(22) Date of filing: 23.10.2007
(51) Int. Cl.: A61B 1/00, A61B 5/07

(54) **CAPSULE TYPE ENDOSCOPE**
KAPSELENDOSKOP
ENDOSCOPE EN CAPSULE

(30) Priority: 08.11.2006 JP 2006303206
(43) Date of publication of application: 19.08.2009
(73) Proprietor: Olympus Corporation, Tokyo 151-0072 (JP)
(72) Inventor: TAMURA, Kazuaki c/o Intellectual Property Support Department, Tokyo 192-8512 (JP); MINAI, Tetsuo c/o Intellectual Property Support Department, Tokyo 192-8512 (JP); OHARA, Jin c/o Intellectual Property Support Department, Tokyo 192-8512 (JP)
(74) Representative: Winter, Brandl, Fürniss, Hübner, Röss, Kaiser, Polte - Partnerschaft
(86) International application number: PCT/JP2007/070980
(87) International publication number: WO 2008/056555

(56) References cited:
- WO-A-2004/068748
- GB-A- 2 148 661
- JP-A- 2006 513 001
- JP-A- 2006 513 670
- US-A1- 2004 092 793

## Description

### Technical Field

The present invention relates to a capsule type endoscope that is introduced into a subject and transmits subject internal information to an external receiving device while moving in the subject with the subject being used as a signal transmission medium.

### Background Art

In the present day, in the field of endoscopes, a capsule type endoscope which is of a swallowable type is in practical use. The capsule type endoscope has an imaging function and a wireless communication function. Further, after the capsule type endoscope is introduced from a mouth of a subject for observation (inspection), it sequentially performs imaging while moving through an organ, e.g., a stomach or a small intestine in accordance with peristaltic motion until it is naturally excreted, thereby acquiring information of the inside of the subject.

Subject internal information (image data) obtained while the capsule type endoscope moves in a body cavity is sequentially transmitted to the outside based on wireless communication, and stored in an externally provided memory. When the subject takes along a receiver including a wireless communication function and a memory function, he/she can freely move after swallowing the capsule type endoscope until the capsule type endoscope is excreted.

Furthermore, as a wireless communication mode of the capsule type endoscope, a human body communication mode of utilizing a subject as a signal transmission medium to transmit image data to the outside has been proposed. For example, in JP-A 2006-513670 (KOKAI), two signal electrodes are provided on an outer peripheral surface of a capsule type endoscope, and a potential difference according to image data is produced between the two electrodes to flow a current through a subject. Moreover, this current is received by receiving electrodes in an external receiving device arranged outside the subject to detect an amount of the current flowing between the receiving electrodes, thereby transmitting the image data by utilizing a human body as a signal transmission medium.

Here, in the capsule type endoscope utilizing the conventional human body communication mode, static electricity from the outside may possibly adversely affect operations of internal circuits. That is, the capsule type endoscope utilizing the conventional human body communication mode has a configuration where transmitting electrodes are arranged on an outer surface. Therefore, as compared with other capsule type endoscopes adopting different communication modes, e.g., a wireless communication mode, it can be considered that static electricity from the outside is apt to be directly received by the transmitting electrodes and the internal circuits of the capsule type endoscope are thereby readily affected. On the same theme, in JP-A 2006-513670 (KOKAI), since a clear countermeasure concerning protection of the internal circuits against static electricity is not taken, and hence operations of the internal circuits may be possibly affected due to an influence of static electricity charging the human body when the capsule type endoscope is applied to the subject.

### Disclosure of Invention

It is an object of the present invention to provide a capsule type endoscope that can suppress an influence of static electricity generated in a plurality of transmitting electrodes in the capsule type endoscope on internal circuits.

According to a first aspect of the present invention, there is provided a capsule type endoscope that is introduced into a subject, acquires information of the inside of the subject while moving, and transmits the acquired information of the inside of the subject to the outside of the subject, comprising: a plurality of transmitting electrodes that are arranged on an outer peripheral surface of the capsule type endoscope to transmit the information of the inside of the subject to the outside of the subject; and a static protecting section that is connected with the plurality of transmitting electrodes and limits a voltage that is generated in the transmitting electrodes to a value equal to or higher than a threshold value.

### Brief Description of Drawings

FIG. 1 is a schematic view showing an entire structure of a capsule type endoscopic system using a capsule type endoscope according to a first embodiment of the present invention;
FIG. 2 is a block diagram showing a detailed structure of the inside of the capsule type endoscope;
FIG. 3 is a circuit block diagram of a matching circuit and an electrostatic protection circuit in the first embodiment according to the present invention;
FIG. 4 is a cross-sectional view when the circuits depicted in FIG. 3 are arranged in the capsule type endoscope;
FIG. 5 is a cross-sectional view when the circuits depicted in FIG. 3 are arranged in a capsule type endoscope in a modification of the first embodiment according to the present invention; and
FIG. 6 is a circuit block diagram of a matching circuit and an electrostatic protection circuit in a second embodiment according to the present invention.

### Best Mode for Carrying Out the Invention

Embodiments according to the present invention will now be explained hereinafter with reference to the accompanying drawings.

### [First Embodiment]

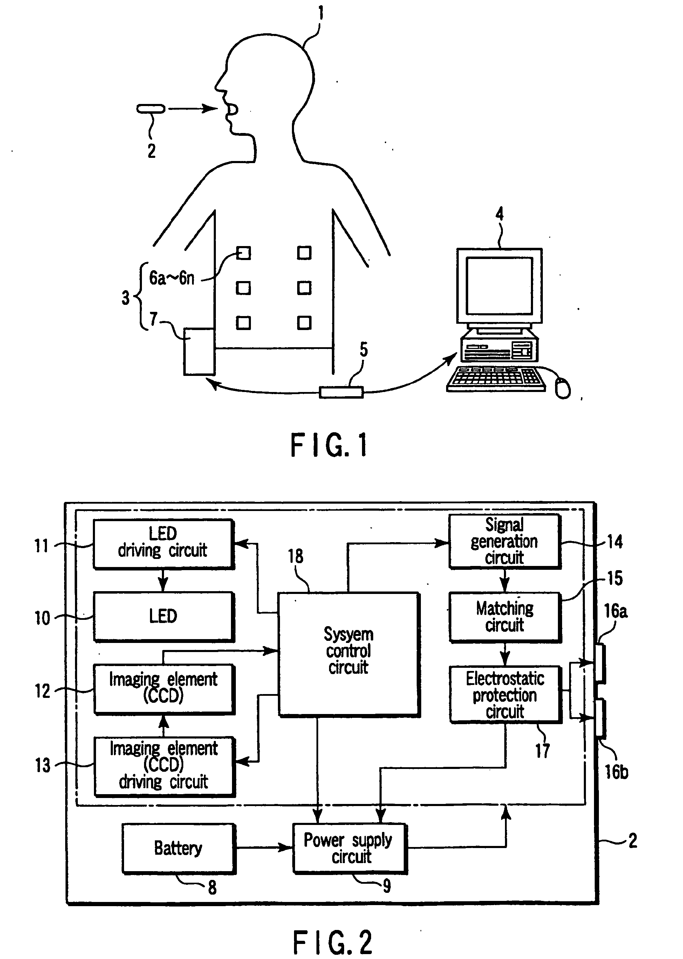

FIG. 1 is a schematic view showing an entire structure of a capsule type endoscopic system using a capsule type endoscope according to a first embodiment of the present invention. The capsule type endoscopic system depicted in FIG. 1 includes a capsule type endoscope 2, a receiving device 3, a display device 4, and a portable recording medium 5.

The capsule type endoscope 2 is introduced into a subject 1, repeatedly performs imaging while moving in the subject 1 to acquire subject internal information (e.g., an image of the inside of the subject 1), and transmits a predetermined signal including the acquired subject internal information to the receiving device 3.

The receiving device 3 receives the transmission signal from the capsule type endoscope 2, and derives an image from the received signal. As shown in FIG. 1, the receiving device 3 includes receiving electrodes 6a to 6n and a processing device 7. The receiving electrodes 6a to 6n are electrodes that are arranged on an outer surface of the subject 1 to receive the transmission signal from the capsule type endoscope 2. The processing device 7 derives an image of the inside of the subject 1 from reception signals from the receiving electrodes 6a to 6n.

The display device 4 displays, e.g., the image of the inside of the subject 1 obtained by the capsule type endoscope 2. The display device 4 is configured as, e.g., a work station that displays an image based on data acquired by the portable recording medium 5. More specifically, the display device 4 has a function of reproducing a video signal from data recorded in the portable recording medium 5 and displaying the reproduced signal in, e.g., a CRT display or a liquid crystal display.

The portable recording medium 5 can be attached to/detached from the processing device 7 and the display device 4, and has a structure enabling outputting and recording information when attached to both the devices. Specifically, the portable recording medium 5 is attached to the processing device 7 to record the subject internal information of the capsule type endoscope 2 while the capsule type endoscope 2 is moving in a body cavity of the subject 1. Moreover, after the capsule type endoscope 2 is discharged from the subject 1, the portable recording medium 5 is removed from the processing device 7 and then attached to the display device 4, and the display device 4 reads the subject internal information recorded in the portable recording medium 5. When data is received/transmitted between the processing device 7 and the display device 4 through the portable recording medium 5, the subject 1 can freely move even if the capsule type endoscope 2 is moving in the subject 1, which differs from a case where the processing device 7 is connected with the display device 4 through a cable.

FIG. 2 is a block diagram showing a detailed structure of the inside of the capsule type endoscope 2. The capsule type endoscope 2 includes a battery 8, a power supply circuit 9, an LED 10, an LED driving circuit 11, an imaging element (a CCD) 12, an imaging element driving circuit 13, a signal generation circuit 14, a matching circuit 15, transmitting electrodes 16a and 16b, an electrostatic protection circuit 17, and a system control circuit 18.

The battery 8 is a power supply for the capsule type endoscope 2. The power supply circuit 9 produces power from the battery 8, and supplies the power to each constituent element in the capsule type endoscope 2. Each constituent element in the capsule type endoscope 2 operates by using the power supplied from the power supply circuit 9 as a driving energy.

The LED 10 is a light source that illuminates an imaging region in the subject 1 when imaging the inside of the subject 1. The LED driving circuit 11 is a driving circuit that drives the LED 10. The imaging element 12 is a CCD type imaging element that images a reflected light image from the imaging region illuminated by the LED 10 to acquire the subject internal information (an image signal). The imaging element driving circuit 13 is a driving circuit that drives the imaging element 12. The image signal acquired by the imaging element 12 is digitized by the system control circuit 18, thereby generating image data of the inside of the subject 1.

The LED and the CCD type imaging element are not absolutely needed as the light source and the imaging element. For example, a CMOS type imaging element may be used as the imaging element.

The signal generation circuit 14 performs processing, e.g., modulation with respect to the image data of the subject 1 acquired by the system control circuit 18, and generates a transmission signal that is used to transmit data to the receiving device 3.

The matching circuit 15 changes a characteristic impedance of the transmission signal generated by the signal generation circuit 14 to perform impedance matching between the transmitting electrodes 16a and 16b and the subject 1. Specifically, the matching circuit 15 includes a structure of varying an impedance of a capacitor component, an inductor component, or a resistor component to change a characteristic impedance of the transmission signal therein. In order to realize this structure, a structure where an impedance varying element is interposed between the transmitting electrodes 16a and 16b in series or in parallel is adopted as the matching circuit 15. Using such a matching circuit 15 enables changing characteristics, e.g., the characteristic impedance of the transmission signal, a power of the transmission signal, a phase of the transmission signal, or a frequency of the transmission signal.

Additionally, the matching circuit 15 includes a current protection resistance element that limits a maximum value of a current flowing in the subject 1.

The transmitting electrodes 16a and 16b are electrodes that transmit the transmission signal output from the matching circuit 15 to the inside of the subject 1. Each of the transmitting electrodes 16a and 16b has electroconductive properties, is formed of a metal that is superior in corrosion resistance and harmless to the human body, and arranged on an outer peripheral surface of the capsule type endoscope 2.

The electrostatic protection circuit 17 suppresses an excess voltage caused due to static electricity generated in the transmitting electrodes 16a and 16b from the outside of the capsule type endoscope 2. One end of the electrostatic protection circuit 17 is connected with the transmitting electrodes 16a and 16b arranged on the capsule outer peripheral surface, and the other end of the same is connected with a GND electrode having a potential equal to a ground (GND) potential generated by the power supply circuit 9. The electrostatic protection circuit 17 connected with the transmitting electrodes 16a and 16b will be explained later.

Here, the electrostatic protection circuit 17 may be connected with not only the transmitting electrodes 16a and 16b but also the power supply circuit 9 of the capsule type endoscope. That is, when the one end of the electrostatic protection circuit 17 is connected with a power supply terminal in the power supply circuit 9 and the other end of the same is connected with the GND electrode, an influence of static electricity generated in the transmitting electrodes 16a and 16b from the outside on the power supply circuit 9 in the capsule type endoscope 2 can be suppressed.

The system control circuit 18 controls operations of the LED driving circuit 11, the imaging element driving circuit 13, the signal generation circuit 14, and the power supply circuit 9, and generates image data of the subject 1 from an image signal obtained by the imaging element 12.

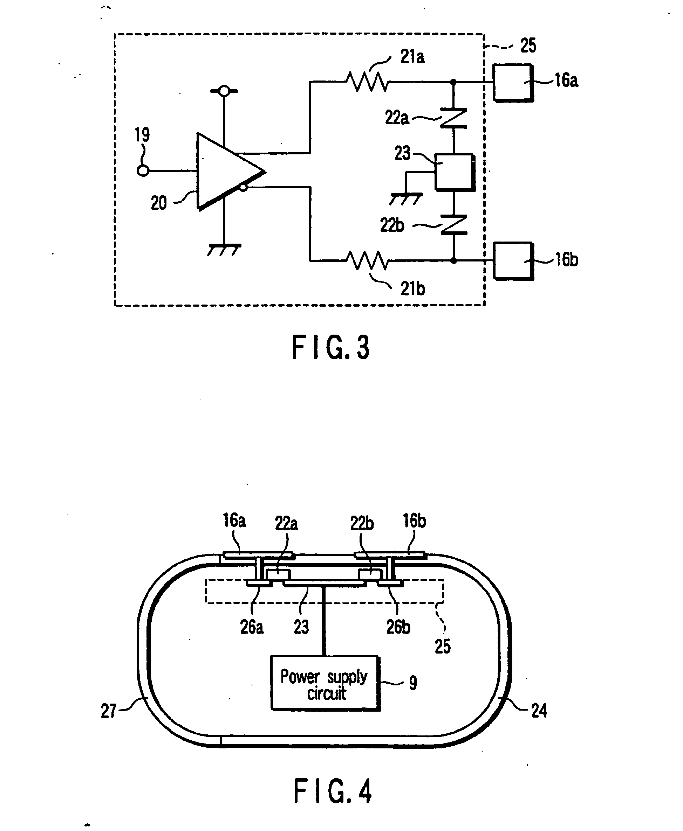

FIG. 3 is a circuit block diagram of the matching circuit 15 and the electrostatic protection circuit 17. Here, in regard to the circuits in FIG. 3, an example where the matching circuit 15 and the electrostatic protection circuit 17 are mounted on a matching circuit substrate 25 is given.

The matching circuit 15 has a port 19, a drive circuit 20, and resistance elements 21a and 21b. An input of the drive circuit 20 is connected with the port 19, and an output of the same is connected with the resistance elements 21a and 21b. The drive circuit 20 performs a buffering operation with respect to a transmission signal input from the signal generation circuit 14 through the port 19. When the transmitting electrode 16a and the transmitting electrode 16b are short-circuited, the resistance elements 21a and 21b limit the amount of current flowing through the transmitting electrode 16a and the transmitting electrode 16b. Each of the resistance elements 21a and 21b is constituted of a resistance element having a resistance value of several-hundred Ω and connected between the drive circuit 20 and the transmitting electrode 16a or 16b in series.

The electrostatic protection circuit 17 is constituted of protection elements 22a and 22b and a GND electrode 23. When static electricity from a human body is applied to the transmitting electrodes 16a and 16b, the protection elements 22a and 22b protect the inner circuits in the capsule type endoscope 2. In the circuits depicted in FIG. 3, each of the protection elements 22a and 22b is formed of a varistor element. One end of the protection element 22a or 22b is connected with the transmitting electrode 16a or 16b, and the other end of the same is connected with the GND electrode 23. The varistor element has a property such that its resistance value is precipitously reduced when a voltage larger than an inherent threshold voltage of the element itself is generated.

The protection element 22a or 22b is not restricted to the varistor element. For example, an element obtained by connecting two zener diodes in an anti-parallel manner may be utilized as the protection element. Further, one zener diode may be utilized to connect a cathode electrode of the zener diode with the transmitting electrode and connect an anode electrode of the same with the GND electrode. Furthermore, the protection element may be a surge absorber element or it may be used the surge absorber element with a capacitor or a resistance element.

According to the structure depicted in FIG. 3, when an excess voltage equal to or larger than a threshold value of the varistor element is generated between the transmitting electrodes due to static electricity, a resistance value of the varistor element is reduced. As a result, flowing a current through the GND electrode 23 from the transmitting electrodes 16a and 16b enables obtaining an effect of preventing the excess voltage from being applied to the inner circuits in the capsule type endoscope 2.

FIG. 4 is a cross-sectional view when the circuits depicted in FIG. 3 are arranged in the capsule type endoscope 2. As shown in FIG. 4, the capsule type endoscope 2 has a structure where the respective circuits depicted in FIG. 1 are arranged in a capsule type housing obtained by combining a capsule case portion 24 with an imaging dome portion 27. Here, in FIG. 4, some of the circuits depicted in FIG. 1 are omitted for clarity.

The imaging dome portion 27 is formed of a transparent material so that an outer part thereof can be illuminated by the LED 10 and light from the outside can be received by the imaging element 12. Moreover, the transmitting electrodes 16a and 16b are arranged on an outer peripheral surface of the capsule case portion 24 formed of an insulator, and the transmitting electrode 16a is insulated from the transmitting electrode 16b by the capsule case portion 24. Additionally, transmitting pads 26a and 26b as output portions of the matching circuit substrate 25 are connected with the transmitting electrodes 16a and 16b, and transmission signals are supplied from the transmitting pads 26a and 26b.

Further, as shown in FIG. 4, the protection elements 22a and 22b are connected with the transmitting electrodes 16a and 16b through the transmitting pads 26a and 26b, and also connected with the GND electrode 23 set to a potential equal to the GND potential produced by the power supply circuit 9 in the capsule type endoscope. Furthermore, although not shown in FIG. 4, a power supply pad that receives a power supply voltage fed from the power supply circuit 9, elements (the resistance elements 21a and 21b) having a matching circuit function, wiring lines, and others are also provided on the matching circuit substrate 25.

Here, as shown in FIG. 4, it is preferable to arrange the matching circuit substrate 25 in close proximity to a facet of the capsule case portion 24 rather than the center of the capsule case portion 24 in the capsule type endoscope 2. This structure shortens lengths of wiring lines between the transmitting electrodes 16a and 16b and the matching circuit substrate 25 and suppresses discharge in the wiring lines between the transmitting electrodes 16a and 16b and the matching circuit substrate 25 when transmitting the transmission signals. Moreover, it is also preferable to arrange the protection elements 22a and 22b on the matching circuit substrate 25 in such a manner that they are arranged near the transmitting electrodes 16a and 16b. This structure likewise suppresses discharge between the protection elements 22a and 22b and the transmitting electrodes 16a and 16b.

When the respective circuits or elements are arranged as shown in FIG. 4, the matching circuit substrate 25 and the protection element 22a can be arranged near the transmitting electrodes 16a and 16b to realize a static protecting function.

As explained above, according to the first embodiment, connecting the protection elements 22a and 22b with the transmitting electrodes 16a and 16b arranged on the outer peripheral surface of the capsule type endoscope 2 enables protecting the inner circuits in the capsule type endoscope 2 against static electricity. In more detail, when the protection elements 22a and 22b appropriately arranged on the matching circuit substrate 25 in the capsule type endoscope 2 are used with respect to the transmitting electrodes 16a and 16b arranged on the outer peripheral surface of the capsule type endoscope 2, an excess voltage caused due to static electricity near the transmitting electrodes 16a and 16b can be suppressed. Additionally, as shown in FIG. 4, when the protection elements 22a and 22b are arranged near the transmitting electrodes 16a and 16b, the phenomenon of discharge of unnecessary static electricity can be suppressed.

### [Modification]

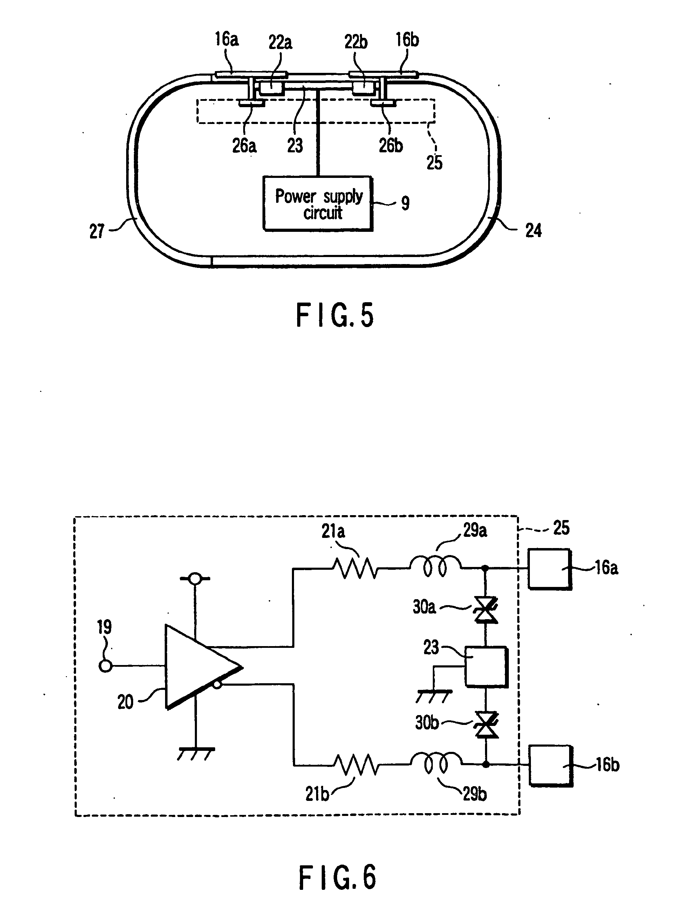

FIG. 5 is a cross-sectional view when the circuits depicted in FIG. 3 are arranged in a capsule type endoscope in a modification of the first embodiment According to the present invention. Here, a capsule type endoscope 2 is constituted of a capsule type housing obtained by combining a capsule case portion 24 with an imaging dome portion 27, and transmitting electrodes 16a and 16b are provided on an outer peripheral surface of the capsule type endoscope 2, as in FIG. 4.

In the modification, a GND electrode 23 is provided on an inner peripheral surface of the capsule case portion 24, and protection elements 22a and 22b are connected with the GND electrode 23. Further, the protection elements 22a and 22b are connected with transmitting electrodes 16a and 16b, respectively. Furthermore, the transmitting electrodes 16a and 16b are connected with transmitting pads 26a and 26b of a matching circuit substrate 25, respectively.

As shown in FIG. 5, the protection elements 22a and 22b are arranged between the transmitting electrodes 16a and 16b and the GND electrode 23 in contact with an inner peripheral surface of the capsule case portion 24 in such a manner that lengths of wiring lines are minimized. Here, although the only one GND electrode 23 provided on the inner peripheral surface of the capsule case portion 24 is shown in FIG. 5, the number of the GND electrode 23 is not restricted to one. For example, the GND electrodes may be separately provided for the protection element 22a and the protection element 22b.

According to the above-explained modification, the protection elements 22a and 22b can be arranged in close proximity to not only the transmitting electrodes 16a and 16b but also the GND electrode 23. Therefore, when an excess voltage caused due to static electricity is supplied to the transmitting electrodes 16a and 16b, excess voltage protection can be performed immediately near the transmitting electrodes 16a and 16b. At this time, since the excess voltage is transmitted to the GND electrode without using an extra path, an effect of protecting the inner circuits can be improved.

### [Second Embodiment]

A second embodiment will now be explained. FIG. 6 is a circuit block diagram of a matching circuit 15 and an electrostatic protection circuit 17 in a capsule type endoscope according to the second embodiment of the present invention. The second embodiment is an example where a protection element as the electrostatic protection circuit is utilized as a part of the matching circuit. It is to be noted that structures denoted by the same reference numerals have the same functions as those in FIG. 3, thereby omitting a description thereof.

In FIG. 6, one end of a resistance element 21a or 21b is connected with an output of a drive circuit 20, and the other end of the same is connected with an inductor element 29a or 29b that performs impedance matching of a transmitting electrode 16a or 16b and a subject 1. Zener diodes 30a and 30b, which are connected in an anti-parallel manner as protection elements of the electrostatic protection circuit 17, are connected with the inductor elements 29a and 29b, respectively. One end of the zener diode 30a or 30b is connected with a GND electrode 23.

The zener diodes 30a and 30b have a function of preventing an excess voltage equal to or higher than a threshold voltage (a zener voltage) from being applied to capsule inner circuits when the excess voltage is applied to the transmitting electrodes 16 and 16b due to static electricity. Further, the zener diodes 30a and 30b have a function of operating as elements each having an inherent capacitance value when a voltage equal to or higher than a threshold value is not applied.

That is, each of the zener diodes 30a and 30b operates as the electrostatic protection element when an excess voltage due to static electricity is applied through each of the transmitting electrodes 16a and 16b, and operates as a capacitor element when an excess voltage is not applied through each of the transmitting electrodes 16a and 16b. At this time, the zener diodes 30a and 30b and the inductor elements 29a and 29b operate together to perform impedance matching of the transmitting electrodes 16a and 16b and the subject 1.

Here, in FIG. 6, the inductor elements 29a and 29b depicted as the elements that perform impedance matching of the transmitting electrodes 16a and 16b and the subject 1 can be constituted of capacitor elements. Further, in regard to a connection method, the inductor elements 29a and 29b can be connected with the transmitting electrodes 16a and 16b not only in series but also in parallel.

It is to be noted that the protection elements are formed of the zener diodes 30a and 30b in the second embodiment, but the protection elements may be constituted of the varistor elements or any other surge absorber elements explained in the first embodiment.

Moreover, although positions of a matching circuit substrate on which the circuits depicted in FIG. 6 are mounted and the protection elements are not restricted in particular in the second embodiment, it is preferable to arrange these members near the transmitting electrodes, as explained in the first embodiment and its modification.

As explained above, according to the second embodiment, properties that the electrostatic protection elements operate as the capacitor elements when a voltage equal to or higher than a threshold voltage is not applied are utilized, the protection elements are enabled to function as the impedance matching elements for the transmitting electrode and the subject when static electricity is not applied, and the protection elements can function as the electrostatic protection elements when static electricity is applied.

## Claims

1. A capsule type endoscope (2) that is introduced into a subject, acquires information of the inside of the subject while moving, and transmits the acquired information of the inside of the subject to the outside of the subject, comprising:
a plurality of transmitting electrodes (16a,16b) that are arranged on an outer peripheral surface of the capsule type endoscope to transmit the information of the inside of the subject to the outside of the subject; and **characterised by** :
a static electricity protecting section (17) that is connected with the plurality of transmitting electrodes and limits a voltage that is generated in the transmitting electrodes to a value equal to or higher than a threshold value.

2. The capsule type endoscope according to claim 1, wherein the static electricity protecting section (17) is connected between the transmitting electrodes and a ground electrode arranged on an inner side of the capsule type endoscope.

3. The capsule type endoscope according to claim 2, wherein the static electricity protecting section (17) is arranged near the transmitting electrodes.

4. The capsule type endoscope according to claim 1, wherein the static electricity protecting section (17) operates as a part of a matching circuit when the voltage equal to or higher than the threshold value is not generated in the transmitting electrodes.

5. The capsule type endoscope according to claim 2, wherein the static electricity protecting section (17) operates as a part of a matching circuit when the voltage equal to or higher than the threshold value is not generated in the transmitting electrodes.

6. The capsule type endoscope according to claim 3, wherein the static electricity protecting section (17) operates as a part of a matching circuit when the voltage equal to or higher than the threshold value is not generated in the transmitting electrodes.

## Patentansprüche

1. Ein Kapselendoskop (2), welches in ein Objekt eingebracht wird, Informationen vom Inneren des Objekts erlangt, während es sich bewegt und die erlangten Informationen des Inneren des Objekts zur Außenseite des Objekts hin überträgt, aufweisend:
eine Mehrzahl von Übertragungselektroden (16a, 16b), die an einer äußeren Umfangsoberfläche des Kapselendoskops angeordnet sind, um die Informationen vom Inneren des Objekts zur Außenseite des Objekts hin zu übertragen, **gekennzeichnet durch**:
einen Schutzabschnitt (17) gegen statische Elektrizität, der mit der Mehrzahl von Übertragungselektroden verbunden ist und eine Spannung, die in den Übertragungselektroden erzeugt wird, auf einen Wert gleich oder höher als einen Schwellenwert begrenzt.

2. Das Kapselendoskop nach Anspruch 1, wobei der Schutzabschnitt (17) gegen statische Elektrizität zwischen die Übertragungselektroden und eine Masseelektrode geschaltet ist, die an einer Innenseite des Kapselendoskops angeordnet ist.

3. Das Kapselendoskop nach Anspruch 2, wobei der Schutzabschnitt (17) gegen statische Elektrizität nahe der Übertragungselektroden angeordnet ist.

4. Das Kapselendoskop nach Anspruch 1, wobei der Schutzabschnitt (17) gegen statische Elektrizität als ein Teil eines Anpassungsschaltkreises arbeitet, wenn die Spannung gleich oder größer als der Schwellenwert nicht in den Übertragungselektroden erzeugt wird.

5. Das Kapselendoskop nach Anspruch 2, wobei der Schutzabschnitt (17) gegen statische Elektrizität als ein Teil eines Anpassungsschaltkreises arbeitet, wenn die Spannung gleich oder größer als der Schwellenwert nicht in den Übertragungselektroden erzeugt wird.

6. Das Kapselendoskop nach Anspruch 3, wobei der Schutzabschnitt (17) gegen statische Elektrizität als ein Teil eines Anpassungsschaltkreises arbeitet, wenn die Spannung gleich oder größer als der Schwellenwert nicht in den Übertragungselektroden erzeugt wird.

## Revendications

1. Endoscope de type capsule (2) qui est introduit dans un sujet, acquière des informations de l'intérieur du sujet tout en bougeant, et transmet les informations acquises de l'intérieur du sujet vers l'extérieur du sujet, comprenant :
- une pluralité d'électrodes de transmission (16a, 16b) qui sont agencées sur une surface périphérique externe de l'endoscope de type capsule pour transmettre les informations de l'intérieur du sujet vers l'extérieur du sujet
**caractérisé par** :
- une section de protection élastique statique (17) qui est reliée avec la pluralité d'électrodes de transmission et limite un voltage qui est généré dans les électrodes de transmission à une valeur égale à ou supérieure à une valeur seuil.

2. Endoscope de type capsule selon la revendication 1, dans lequel la section élastique de protection statique (17) est reliée entre les électrodes de transmission et l'électrode de masse agencée sur un côté intérieur de la capsule de type endoscope.

3. Endoscope de type capsule selon la revendication 2, dans lequel la section élastique de protection statique (17) est agencée à proximité des électrodes de transmission.

4. Endoscope de type capsule selon la revendication 1, dans lequel la section élastique de protection statique (17) fonctionne comme une partie d'un circuit d'adaptation lorsque le voltage égal ou supérieur à la valeur seuil n'est généré dans les électrodes de transmission.

5. Endoscope de type capsule selon la revendication 2, dans lequel la section élastique de protection statique (17) fonctionne comme une partie d'un circuit d'adaptation lorsque le voltage égal ou supérieur à la valeur seuil n'est pas généré dans les électrodes de transmission.

6. Endoscope de type capsule selon la revendication 3, dans lequel la section élastique de protection statique (17) fonctionne comme une partie d'un circuit d'adaptation lorsque le voltage égal ou supérieur à la valeur seuil n'est pas généré dans les électrodes de transmission.
